(19) Europäisches Patentamt
European Patent Office
Office européen des brevets

(11) Numéro de publication: **0 137 839 B1**

# (12) FASCICULE DE BREVET EUROPEEN

(45) Date de publication du fascicule du brevet: **27.01.88**

(51) Int. Cl.⁴: **A 61 L 15/00**

(21) Numéro de dépôt: **84901358.6**

(22) Date de dépôt: **20.03.84**

(86) Numéro de dépôt international: **PCT/FR 84/00074**

(87) Numéro de publication internationale: **WO 84/03631 (27.09.84** Gazette **84/23)**

## (54) MATERIAU ABSORBANT COMPORTANT UN DERIVE ISOTHIAZOLINE-ONE-3, APPLICATION A L'HYGIENE CORPORELLE ET PROCEDE D'OBTENTION DE CELUI-CI.

(30) Priorité: **23.03.83 FR 8304785**

(43) Date de publication de la demande: **24.04.85 Bulletin 85/17**

(45) Mention de la délivrance du brevet: **27.01.88 Bulletin 88/4**

(84) Etats contractants désignés: **BE CH DE GB LI LU NL SE**

(56) Documents cités:
**GB - A - 2 083 748**
**US - A - 4 248 685**

(73) Titulaire: **BEGHIN-SAY SOCIETE ANONYME, F-59239 Thumeries (FR)**

(72) Inventeur: **EHRET, Philippe, 130, avenue d'Alsace, F-68000 Colmar (FR)**

(74) Mandataire: **Quéré, Jean Pierre, BEGHIN-SAY Service Propriété Industrielle 54, Avenue Hoche, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

L'invention concerne principalement le domine de l'hygiéne corporelle.

Elle a pour objet un matériau absorbant constitué d'un polyélectrolyte hydrocolloïdal insoluble dans l'eau et absorbant plusieurs fois son poids en liquide aqueux.

Elle a également pour objet une application de ce matériau et un procédé d'obtention de celui-ci.

Etat de la technique

On sait que l'urine, stérile à l'origine (sauf en cas d'infection urinaire) est contaminée, dès sa sortie de l'urètre, par la flore du périnée qui est le siège d'un grand nombre de bactéries dont 80% sont formées par:

Protéus (50%), Klebsiella, Pseudomonas, Coli.

L'urine est, en outre, un excellent substrat de croissance pour ces germes.

De même le sang menstruel qui contient des protéines, diverses amines et des lipides, constitue un milieu de culture pour les bactéries citées plus haut.

Ainsi l'urée est transformée en ammoniac, grâce aux uréases que possèdent certaines bactéries, telles que Proteus et Klebsiella, selon la réaction suivante:

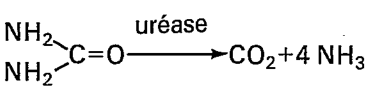


(urée)

Les acides aminés constituant les protéines subissent également une dégradation qui entraine la formation d'ammoniac.

D'autres corps, comme les lipides du sang, sont également transformés en d'autres produits de dégradation selon le schéma suivant:

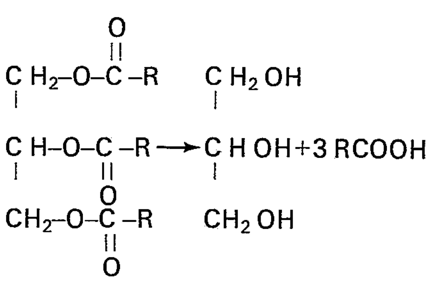

Triglycérides Glycérol

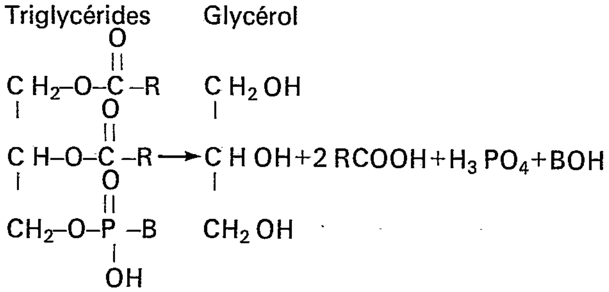

Phospholipides Glycérol

R = Dérivés d'acides gras
B = Fonction alcool amino

Les acides gras formés sont entre autre l'acide butyrique, isobutyrique et isovalérique.

Toutes ces réactions résultant de l'activité des bactéries ont comme caractéristique commune de conduire à des composés d'odeur très désagréable.

Chez les individus en état d'incontinence urinaire et/ou fécale et chez les femmes en période menstruelle, cette flore du périnée est directement en contact avec les fluides corporels absorbés par le tampon absorbant, ce qui entraine la formation de mauvaises odeurs dues à l'activité des bactéries sur ces fluides. On conçoit que ce phénomène, à la limite tolérable quand il s'agit d'un bébé, devient psychologiquement pénible à supporter pour un adulte.

Afin d'éliminer cet inconvénient, il a été proposé d'associer un bactéricide aux matelas absorbants des couches et serviettes périodiques.

Ainsi le brevet français FR-A-2 490 093 (Landstingens inkopscentrallic ekonomisk forening) décrit l'incorporation, de préférence à la surface, d'un sel de cuivre hydrosoluble dans un matelas absorbant formé de fibres de cellulose. Le cuivre, sous forme ionique, est en effet connu pour ses activités bactéricides et fongicides.

Cette technique présente, toutefois, certains inconvénients. Le bactéricide déposé sur le matelas est agressif vis à vis de la peau de l'usager: cela peut entraîner certains accidents dermatologiques et même affaiblir les défenses naturelles.

La mise en œuvre industrielle du procédé présente, en outre, certaines difficultés car il est nécessaire de traiter toute la surface du matelas absorbant.

De plus, les procédés de dépôt (pulvérisation, soupoudrage) présentent un danger non négligeable pour le personnel.

La demande de brevet européen EP-A-19 371 (Unilever) propose d'incorporer un ion d'un métal de transition (tels que le cuivre ou le zinc) dans un additif à rétention amélioré (ARA) qui est, comme son nom l'indique, un corps incorporé au matelas absorbant afin d'améliorer ses capacités d'absorption. Il est établi une liaison ionique entre les groupes anioniques (notamment COO) et l'ion du métal de transition. Cette liaison est suffisamment labile pour permettre à l'ion du métal de transition de migrer hors de l'ARA dans le tampon et provoquer la coagulation des grosses protéines. Afin d'obtenir la migration de ces ions hors de l'ARA il est, toutefois, nécessaire d'en incorporer une quantité relativement importante puisque environ 80% des groupes COOH sont neutralisés. Ceci présente l'inconvénient de limiter la vitesse de prise en gel de l'ARA et de diminuer ses capacités d'absorption.

On sait de plus que les métaux de transition comme le cuivre ou le zinc sont des bactéricides, donc toxiques, et que toxicologiquement parlant il est préférable que ceux-ci soient présents à des doses très faibles.

L'objet de l'invention est de proposer un superabsorbant présentant une activité bactéricide pouvant être communiquée à son environnement constitué par le matelas absorbant. Ce qui présente comme avantage d'éliminer les risques d'irritation de la peau avant l'arrivée du liquide nutriment.

Dans le langage courant le terme «superabsorbant» est synonyme d'additif à rétention amélioré ou de matériau absorbant plusieurs fois son poids en liquide.

Description générale de l'invention

L'invention est caractérisée en ce que le matériau absorbant comporte au moins un dérivé isothiazoline – one – 3 de formule générale:

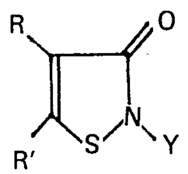

Dans laquelle:

Y est un atome d'hydrogène, un groupe alkyle en $C_1$ à $C_8$ linéaire ou ramifié, un groupe cycloalkyle en $C_3$ à $C_6$,

R est un atome d'hydrogène, un groupe alkyle en $C_1$ à $C_4$ ou un halogène,

R' est un atome d'hydrogène, un groupe alkyle en $C_1$ à $C_4$ ou un halogène.

En ce qui concerne l'application, l'invention est caractérisée en ce que le matériau absorbant est utilisé comme additif à rétention amélioré, incorporé dans les matelas absorbants destinés à l'hygiène corporelle, notamment, ceux destinés aux couches pour bébés ou pour adultes incontinents, ou aux protections périodiques.

En ce qui concerne le procédé d'obtention du matériau, l'invention est caractérisée en ce que, dans un mélange eau-alcool, on ajoute dans un premier temps au moins un dérivé isothiazoline – one – 3 puis le polyélectrolyte hydrocolloïdal, le mélange étant ensuite filtré.

Polyélectrolytes – Hydrocolloïdaux

Les polyélectrolytes hydrocolloïdaux insolubles dans l'eau et absorbant plusieurs fois leur poids en liquide aqueux peuvent être divisés en deux grandes catégories à savoir les polyélectrolytes naturels d'une part et les polyélectrolytes synthétiques d'autre part (voir notamment US 4 043 952).

1) Les polyélectrolytes naturels sont avantageusement choisis parmi les dérivés anioniques de l'amidon ou de la cellulose ou du dextrane encore appellés polysaccharides. Parmi les groupements anioniques on peut citer les groupements carboxyle, sulfonate, sulfate ou phosphate. Les groupements anioniques préférés sont les groupements carboxylalkyle et notamment les groupements carboxyéthyle et carboxyméthyle.

Ces groupements anioniques sont de préférence neutralisés par un cation alcalin tel que le sodium ou par une amine primaire, secondaire ou tertiaire dans une proportion supérieure à 40% par rapport au nombre total de groupes ioniques et de préférence comprise entre 40 et 85%. Ceci, de manière bien connue, améliore la capacité d'absorption des polymères.

Ces polyélectrolytes sont sous forme réticulée de manière à les rendre insolubles dans l'eau mais évidemment sans altérer leur capacité d'absorption.

Cette réticulation peut résulter de la formation de liaisons covalentes au moyen d'estérification ou d'éthérification qui peuvent être assurées par des diols, des dihalogénures, des épichlorhydrines comme dans EP-19 371 (Unilever NV).

La réticulation peut également être assurée par des métaux de transition appartenant aux groupes suivants de la classification périodique: III B, IV B, V B, VI B, VII B, VIII B, III A, IV A, V A, VI A. Parmi ceux-ci on peut citer l'aluminium, le zirconium, le chrome, le titane, le zinc.

2) Les homopolymères ou copolymères des acides carboxyliques insaturés comme les acides polyacryliques ou méthacryliques (voir notamment US 4 043 952) ou les homopolymères ou copolymères contenant des acides sulfoniques comme ceux provenant de la polymérisation d'acides sulfoniques insaturés.

Ces homopolymères ou copolymères sont également sous forme anionique, partiellement ou totalement, et sont réticulés par les mêmes moyens que décrits dans 1).

Les polymères préférés sont ceux comportant les groupes carboxyles et spécialement l'acide polyacrylique ou polyméthacrylique.

Tous ces polymères absorbent entre cinq et plusieurs centaines de fois leur poids en liquide aqueux selon qu'il s'agit d'eau, d'urine, de sang, y compris le sang menstruel.

A titre indicatif, les méthodes comme celles décrites dans les brevets DE-2 702 781 ou FR-2 305 452 peuvent être utilisées pour déterminer le degré d'absorption.

Un autre paramètre important pour ces polymères réside dans leur vitesse de prise en gel. Un bon polymère doit présenter un temps de prise en gel le plus faible possible en se référant par exemple au test du Vortex. (G. Goldstein et M. Pierre, Marketing Technology Serivce Insight 81, Section IX-1-18, Publication Miller Freeman.).

Dérivé isothiazoline – one – 3

Les dérivés qui sont décrits par la formule générale sont connus en soi ainsi que leur activité bactéricide.

La brevetabilité de l'invention ne réside pas dans cette activité biocide déjà connue mais dans la faculté que ces composés révèlent de pouvoir migrer hors du polyélectrolyte hydrocolloïdal et de gêner, sinon d'empêcher, le développement des bactéries dans tout le matelas absorbant et

ainsi d'empêcher la formation de mauvaises odeurs.

Les brevets suivants décrivent ces dérivés ainsi que leur procédé de préparation:

* FR 2 139 421 où l'on trouvera une liste exhaustive des dérivés isothiazoline - one - 3.

* US 3 517 022 où l'on trouvera la préparation et les propriétés biocides des dérivés isothiazoline - one - 3.

* et également US 3 544 480, US 3 761 488, FR 2 398 505.

Parmi tous les dérivés qui répondent à la formule générale, on préférera les dérivés présentant une formule dans laquelle:

Y est un atome d'hydrogène ou un groupe alkyle en $C_1$ à $C_4$

R est un atome d'hydrogène ou un groupe alkyle en $C_1$ à $C_4$

R' a la même signification que précédemment.

Et, de préférence, ceux présentant une formule générale dans laquelle:

Y est un groupement méthyle

R est un atome d'hydrogène

R' est un atome d'hydrogène ou un halogène ou un groupe méthyle.

L'halogène peut être, le chlore ou le brome. De manière à avoir une activité bactéricide améliorée tout en conservant les capacités migrantes propres à ces dérivés il est souhaitable que le matériau absorbant comporte au moins deux dérivés l'un (A) répondant à la formule générale dans laquelle R' est un halogène, tel que le chlore, et de préférence répondant à la formule suivante:

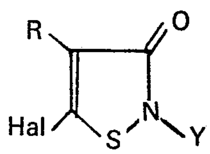

R étant un atome d'hydrogène ou un groupe alkyle en $C_1$ à $C_4$

Y étant un atome d'hydrogène ou un groupe alkyle en $C_1$ à $C_4$

Hal étant de préférence le chlore.

L'autre (B) répondant à la formule générale dans laquelle R' est un atome d'hydrogène, un groupe alkyle en $C_1$ à $C_4$ et, de préférence, répondant à la formule suivante:

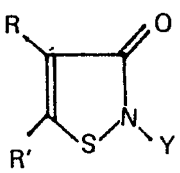

R' étant un atome d'hydrogène ou un groupe alkyle en $C_1$ à $C_4$

Y étant un atome d'hydrogène ou un groupe alkyle en $C_1$ à $C_4$

R étant un atome d'hydrogène ou un groupe alkyle en $C_1$ à $C_4$

De préférence le matériau absorbant comporte les deux dérivés suivants:

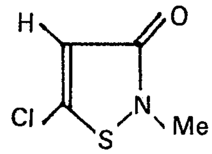

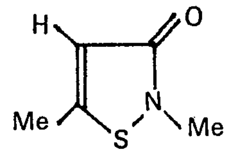

Connus sous les dénominations officielles suivantes:

A – chloro - 5 - méthyl - 2 - isothiazoline - one - 3

B – méthyl - 5 - méthyl - 2 - isothiazoline - one - 3

Le mélange de ces produits est vendu par la Société Américaine ROHM & HAAS sous la marque KATHON.C.G.(R) qui comporte 1,5% environ de dérivés isothiazoline - one - 3 (1,2% de A et 0,3% de B) et 98,5% de matière inerte en poids.

De préférence, pour les matériaux absorbants qui comportent les dérivés (A) et (B), le rapport molaire entre ces deux composés est compris entre 0,5 et 8.

Bien qu'il soit possible d'incorporer le dérivé isothiazoline - one - 3 dans une fourchette étendue de proportion en poids par rapport au polyélectrolyte sans sortir de l'invention, il est préférable que le matériau absorbant comporte une proportion en poids de dérivé isothiazoline - one - 3 par rapport au polyélectrolyte comprise entre 1,5 et 750 ppm et de préférence entre 7,5 et 450 ppm et encore mieux de 10,5 à 150 ppm.

Comme cela a été écrit plus haut et c'est ce qui fait l'intérêt de l'invention, les dérivés isothiazoline - one - 3 migrent à l'extérieur du polyélectrolyte. Il a été trouvé qu'il était tout à fait possible et même souhaitable que le matériau comporte également un ion métallique d'un sel de métal de transition connu pour ses activités bactéricides et coagulantes, de préférence, le cuivre ou le zinc.

Ces ions peuvent être présents sous forme d'halogénure ou de sels organiques. On en trouvera une description et une énumération exhaustive dans FR 2 490 093.

On préférera, toutefois, les halogénures.

De façon surprenante, ceci permet de diminuer la quantité de dérivé isothiazoline - one - 3 nécessaire, ce qui est très intéressant sur le plan toxicologique, et, par ailleurs, tandis que le dérivé isothiazoline - one - 3 migre, l'ion métallique reste dans le polymère ce qui assure une couverture bactéricide encore améliorée. En outre, la vitesse de prise en gel est augmentée.

De préférence, le rapport en poids dérivé isothiazoline - one - 3/métal de transition est compris entre 0,012 et 0,018.

Ces matériaux absorbants selon l'invention, lorsqu'ils sont disséminés sous forme de grains ou

de poudre dans les tampons absorbants dans des proportions connues pour permettre une absorption suffisante, éliminent les odeurs dont l'origine a été discutée dans le préambule.

Le matériau absorbant selon l'invention est obtenu à température ambiante de préférence avec un mélange eau/méthanol ou eau/éthanol dans des proportions comprises entre 80/20 et 90/10.

Quand le matériau absorbant comporte un sel d'ion métallique il est préférable d'incorporer celui-ci avant le dérivé isothiazoline - one - 3.

L'introduction du matériau absorbant dans le matelas s'effectue, de manière connue, de plusieurs façons différentes, dont on peut citer:

– Le dépôt continu ou discontinu entre deux nappes de fluff (cellulose défibrée). Une variante particulièrement intéressante de ce procédé est illustrée par la demande de brevet EP-A-3142

– Le mélange avec le fluff.

Ce fluff est ensuite pris en sandwich entre des feuilles d'ouate ou de non tissé.

Exemples

Tous les essais ont été réalisés avec le composé isothiazoline – one – 3, résultant du mélange des deux dérivés suivants:

A – chloro – 5 méthyl – 2 isothiazoline – one – 3
B – méthyl – 5 méthyl – 2 isothiazoline – one – 3
(Kathon CG de la Société Rhom & Haas).

Exemple 1

Procédé général d'obtention du matériau absorbant comportant le kathon CG ainsi que du cuivre sous forme ionique

Dans un mélange méthanol/eau (80/20), on ajoute l'ion métallique sous forme de chlorure cuivrique puis le kathon CG et enfin le poly-électrolyte.

Les proportions sont les suivantes en parties en poids:

| | |
|---|---|
| – polyélectrolyte | 100 |
| – Kathon CG | 1,5 |
| – Cu $Cl_2$ | 3 |

Après filtration on récupère la poudre de polymère associée aux deux autres composés. Le méthanol est recyclé.

Exemple 2

Vitesse de prise en gel

Les trois polyélectrolytes suivants ont été testés:

A amidon polycarboxyle connu sous le nom de marque SANWET de la Société SANYO.

B Polyacrylate d'un métal alcalin connu sous le nom de marque AQUAKEEP de la Société SEITEETTSU.

C Carboxyméthylamidon connu sous le nom de marque AKUCELL de la Société AKZO.

En utilisant le test dit du VORTEX (voir description des polyélectrolytes) on obtient les résultats suivants pour la vitesse de prise en gel (en s):

| | Urine 100 cc | Sang 100 cc |
|---|---|---|
| 2 g de B | 3″ | 26″ |
| avec Kathon CG + $Cu^{++}$ | 2,5″ | 19″ |
| 4 g de C | 19″ | 120″ |
| avec Kathon CG + $Cu^{++}$ | 16,5″ | 57″ |

On constate donc de manière tout a fait surprenante que la présence des deux composés bactéricides améliore sensiblement la vitesse de prise en gel.

Exemple 3

Migration des bactéricides

Méthode de la zone d'inhibition

Des disques de papiers, sur lesquels est déposée une certaine quantité de produit à tester, sont placés sur de l'agar ensemencé avec un microorganisme déterminé (ex: Pseudomones Oleoverans).

Les boîtes de Petri sont placées au réfrigérateur pendant 24 h., puis à l'étuve pendant 18 h. à 30 °C.

Après incubation, on mesure le diamètre total des zones d'inhibition apparues:

| Produits testés<br>Quantités déposées par disque | | Diamètre de la zone d'inhibition | Quantité de Kathon correspondante |
|---|---|---|---|
| B | 3,3 mg | 18 mm | 0 |
| B+$Cu^{++}$ 5% Cu $Cl_2$ | 3,3 mg | 18 mm | 0 |
| | 5 mg | 29 mm | 0,37 g |
| B+ | 10 mg | 34 mm | 0,63 g |
| Kathon CG | 15 mg | 39 mm | 0,96 g |
| | 20 mg | 42 mm | 1,24 g |
| B+ | 5 mg | 28 mm | 0,33 g |
| | 10 mg | 34 mm | 0,63 g |

| Produits testés<br>Quantités déposées par disque | | | Diamètre de la zone d'inhibition | Quantité de Kathon correspondante |
|---|---|---|---|---|
| Kathon CG | | | | |
| $+Cu^{++}$ | 15 | mg | 38 mm | 0,88 g |
| 5% Cu $Cl_2$ | 20 | mg | 40 mm | 1,05 g |

Exemple 4

Mésure de l'intensité de l'odeur

On fait subir à un échantillon de personnes les trois séries de tests suivants:

– détermination de la concentration seuil de détection (C.S) d'un produit odorant (Steiger Chem. Tech. Vol 1 avril 1971).

– Etablissement de la courbe étalon avec divers échantillons renfermant des multiples de la concentration seuil.

– détermination de l'activité du bactéricide par évaluation de l'intensité de l'odeur, celle-ci étant qualifiée en unité C.S.

L'essai selon l'exemple est effectué sur une série de serviettes périodiques contenant l'ARA A avec 0,1 ou 0,15% de Kathon CG et 0,2% de Cu $Cl_2$. Par rapport au poids de la serviette. Un essai comparatif a également été effectué.

On dépose 7 ml de sang de boeuf par serviette et 1 ml de suspension bactérienne préparée en mélangeant:

1 ml d'une culture de 24 h de Pseudomonas aeruginosa

1 ml d'une culture de 24 h de Escherichia Coli

0,8 ml d'une culture de 48 h de Klebsiella Pneumonia

5 ml d'une culture de 48 h de Proteus Mirabilis

– Chaque serviette est ensuite placée dans le flacon en polyéthylène, puis mis en incubation pendant 15 h à 30 °C. La mesure de l'intensité de l'odeur se fait sans les mêmes conditions.

Les résultats sont les suivants:

| | |
|---|---|
| Kathon CG 0,10% | 4 |
| Kathon CG 0,15% | 1 |
| Témoin (sans bactéricides) | 6 |

Exemple 5

Test sur produit fini

Des changes renfermant de l'ARA B (Aquakeep) modifié (5% de $Cu^{2+}$ et 2% de Kathon) ont été portés pendant 12 heures par des incontinents.

Un lot témoin sans ARA modifié a été testé parallèlement.

Après 12 heures, une mesure olfactive et une mesure de pH de l'urine du change ont été faites.

Résultats:

| | ARA non traité | ARA traité |
|---|---|---|
| Nombre de changes testés | 43 | 56 |
| Nombre de changes malodorants | 14 | 0 |
| pH moyen | 8,5 | 7,3 |

La présence de bactéricides sur l'ARA diminue donc de façon considérable, l'apparition de produits malodorants, et limite l'augmentation du pH de l'urine, diminuant ainsi son pouvoir irritant.

## Revendications

1. Matériau absorbant constitué d'un polyélectrolyte hydrocolloïdal insoluble dans l'eau et absorbant plusieurs fois son poids en liquide aqueux, caractérisé en ce qu'il comporte au moins un dérivé isothiazoline-one-3 de formule générale:

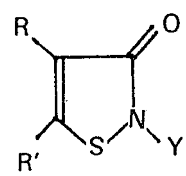

dans laquelle

Y est un atome d'hydrogène, un groupe alkyle en $C_1$ à $C_8$ linéaire ou ramifié, un groupe cycloalkyle en $C_3$ à $C_6$.

R est un atome d'hydrogène, un groupe alkyle en $C_1$ à $C_4$ ou un halogène.

R' est un atome d'hydrogène, un groupe alkyle en $C_1$ à $C_4$ ou un halogène.

2. Matériau absorbant selon la revendication 1, caractérisé en ce que

Y est un atome d'hydrogène ou un groupe alkyle en $C_1$ à $C_4$.

R est un atome d'hydrogène ou un groupe alkyle en $C_1$ à $C_4$.

R' a la même signification que dans la revendication 1.

3. Matériau absorbant selon la revendication 2, caractérisé en ce que

Y est un groupement méthyle

R est un atome d'hydrogène

R' est un atome d'hydrogène ou un groupe méthyle ou un halogène.

4. Matériau absorbant selon l'une des revendications précédentes, caractérisé en ce qu'il comporte au moins deux dérives isothiazoline-one-3,

A – l'un au moins répondant à la formule dans laquelle R' est un halogène.

B – au moins un autre répondant à la formule dans laquelle R' est un atome d'hydrogène, un groupe alkyle en $C_1$ à $C_4$

5. Matériau absorbant selon la revendication 4, caractérisé en ce qu'il comporte les deux dérivés isothiazoline-one-3 suivants:

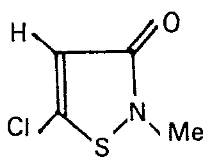

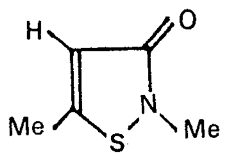

6. Matériau absorbant selon l'une des revendications 4 ou 5, caractérisé en ce que la proportion en mole A/B est comprise entre 0,5 et 8.

7. Matériau absorbant selon l'une des revendications précédentes, caractérisé en ce qu'il comporte le dérivé isothiazoline-one-3 dans une proportion en poids comprise entre 1,5 et 750 ppm par rapport au polyélectrolyte.

8. Matériau absorbant selon la revendication 7, caractérisé en ce qu'il comporte le dérivé isothiazoline-one-3 dans une proportion en poids comprise entre 7,5 et 450 ppm par rapport au polyélectrolyte.

9. Matériau absorbant selon l'une des revendications précédentes, caractérisé en ce que le dérivé isothiazoline-one-3 est associé à un sel d'un métal de transition.

10. Matériau absorbant selon la revendication 9, caractérisé en ce que le métal de transition est choisi parmi le cuivre et le zinc.

11. Matériau absorbant selon l'une des revendications 9 ou 10, caractérisé en ce que le rapport en poids dérivé isothiazoline-one-3/métal de transition est compris entre 0,012 et 0,018.

12. Application du matériau absorbant selon l'une des revendications précédentes, caractérisé en ce qu'il est utilisé comme additif à rétention amélioré, incorporé dans les tampons absorbants pour l'hygiène corporelle, notamment ceux destinés aux couches pour bébés ou pour adultes incontients, ou aux serviettes périodiques.

13. Procédé d'obtention d'un matériau absorbant selon l'une des revendications précédentes caractérisé en ce que dans un mélange eau-alcool dans des proportions comprises entre 80/20 et 90/10, on ajoute dans un premier temps au moins un dérivé isothiazoline-one-3 puis le polyélectrolyte hydrocolloïdal et en ce qu'on filtre ensuite.

14. Procédé d'obtention d'un matériau absorbant selon la revendication 13, caractérisé en ce qu'on ajoute un sel d'un métal de transition avant le polyélectrolyte hydrocolloïdal.

## Patentansprüche

1. Saugfähiges Material, das von einem hydrokolloidalen Polyelectrolyt, der in Wasser unlöslich ist und ein mehrfaches seines Gewichts an wässriger Flüssigkeit zu absorbieren vermag, gebildet wird, dadurch gekennzeichnet, dass es mindestens ein Iso-thiazolin-on-3-Derivat der allgemeinen Formel enthält

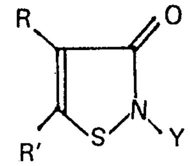

in der bedeuten:

Y ein Wasserstoffatom, eine geradkettige oder verzweigte Alkylgruppe mit 1 bis 8 C-Atomen oder eine Cycloalkylgruppe mit 3 bis 6 C-Atomen;

R ein Wasserstoffatom, eine Alkylgruppe mit 1 bis 4 C-Atomen oder ein Halogen, und

R' ein Wasserstoffatom, eine Alkylgruppe mit 1 bis 4 C-Atomen oder ein Halogen.

2. Saugfähiges Material nach Anspruch 1, dadurch gekennzeichnet, dass in der allgemeinen Formel

Y ein Wasseratom oder eine Alkylgruppe mit 1 bis 4 C-Atomen,

R ein Wasserstoffatom oder eine Alkylgruppe mit 1 bis 4 C-Atomen sind und

R' die gleiche Bedeutung wie in Ansrpuch 1 angegeben hat.

3. Saugfähiges Material nach Anspruch 2, dadurch gekennzeichnet, dass in der allgemeinen Formel

Y eine Methylgruppe,

R ein Wasserstoffatom und

R' ein Wasserstoffatom oder eine Methylgruppe oder ein Halogen bedeuten.

4. Saugfähiges Material nach einem der vorhergehenden Ansprüche, dadurch gekennzeichnet, dass es mindestens zwei Isothiazolin-on-3-Derivate enthält, wovon

A – mindestens eines der allgemeinen Formel entspricht, in der R' ein Halogen ist, und

B – mindestens ein anderes der allgemeinen Formel entspricht, in der R' ein Wasserstoffatom oder eine Alkylgruppe mit 1 bis 4 C-Atomen ist.

5. Saugfähiges Material nach Anspruch 4, dadurch gekennzeichnet, dass es die beiden nachstehenden Isothiazolin-on-3-Derivate enthält:

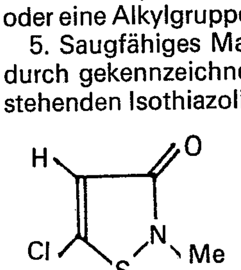

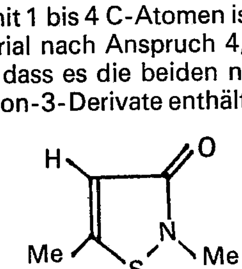

6. Saugfähiges Material nach einem der Ansprüche 4 und 5, dadurch gekennzeichnet, dass das Molverhältnis A/B im Bereich von 0,5 bis 8 liegt.

7. Saugfähiges Material nach einem der vorhergehenden Ansprüche, dadurch gekennzeichnet, dass es das Isothiazolin-on-3-Derivat in einem Gewichtsverhältnis von 1,5 bis 750 ppm, bezogen auf den Polyelektrolyt, enthält.

8. Saugfähiges Material nach Anspruch 7, dadurch gekennzeichnet, dass es das Isothiazolin-on-3-Derivat in einem Gewichtsverhältnis von 7,5 bis 450 ppm, bezogen auf den Polyelektrolyt, enthält.

9. Saugfähiges Material nach einem der vorhergehenden Ansprüche, dadurch gekennzeichnet, dass das Isothiazolin-on-3-Derivat mit einem Salz eines Übergangsmetalls verbunden ist.

10. Saugfähiges Material nach Anspruch 9, dadurch gekennzeichnet, dass das Übergangsmetall Kupfer oder Zink ist.

11. Saugfähiges Material nach Anspruch 9 oder 10, dadurch gekennzeichnet, dass das Gewichtsverhältnis von dem Isothiazolin-on-3-Derivat zu dem Übergangsmetall im Bereich von 0,012 bis 0,018 liegt.

12. Verwendung des saugfähigen Materials nach einem der vorhergehenden Ansprüche, dadurch gekennzeichnet, dass es als Additiv zur Verbesserung der Retention benutzt wird, eingearbeitet in saugfähige Tampons für Körperhygiene, insbesondere solche, die für Windeln für Säuglinge oder an Inkontinenz leidende Erwachsene oder für Damenbinden bestimmt sind.

13. Verfahren zur Herstellung eines saugfähigen Materials nach einem der vorhergehenden Ansprüche, dadurch gekennzeichnet, dass man einem Gemisch aus Wasser und Alkohol im Verhältnis von 80:20 bis 90:10 zu einem ersten Zeitpunkt mindestens ein Isothiazolin-on-3-Derivat, dann den hydrocolloïdalen Polyelektrolyt zugibt und danach das Ganze filtriert.

14. Verfahren nach Anspruch 13, dadurch gekennzeichnet, dass man vor dem hydrokolloïdalen Polyelektrolyt ein Salz eines Übergangsmetalls zufügt.

**Claims**

1. Absorbent material consisting of a hydrocolloidal polyelectrolyte which is insoluble in water and absorbs several times its weight of aqueous liquid, characterised in that it contains at least one isothiazolin-3-one derivative of the general formula:

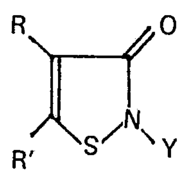

in which

Y is a hydrogen atom, a linear or branched $C_1$ to $C_8$ alkyl group or a $C_3$ to $C_6$ cycloalkyl group.

R ist a hydrogen atom, a $C_1$ to $C_4$ alkyl group or a halogen.

R' is a hydrogen atom, a $C_1$ to $C_4$ alkyl group or a halogen.

2. Absorbent material according to claim 1, characterised in that

Y is a hydrogen atom or a $C_1$ to $C_4$ alkyl group.

R is a hydrogen atom or a $C_1$ to $C_4$ alkyl group.

R' has the same meaning as in claim 1.

3. Absorbent material according to claim 2, characterised in that

Y is a methyl group

R is a hydrogen atom

R' is a hydrogen atom, a methyl group or a halogen.

4. Absorbent material according to one of the preceding claims, characterised in that it contains at least two isothiazolin-3-one derivatives,

A – at least one corresponding to the formula in which R' is a halogen.

B – at least another corresponding to the formula in which R' is a hydrogen atom or a $C_1$ to $C_4$ alkyl group.

5. Absorbent material according to claim 4, characterised in that it contains the following two isothiazolin-3-one derivatives:

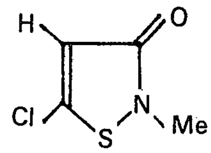

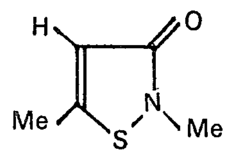

6. Absorbent material according to one of claims 4 or 5, characterised in that the molar ratio of A/B is between 0.5 and 8.

7. Absorbent material according to one of the preceding claims, characterised in that it contains the isothiazolin-3-one derivative in an amount by weight of between 1.5 and 750 ppm, with respect to the polyelectrolyte.

8. Absorbent material according to claim 7, characterised in that it contains the isothiazolin-3-one derivative in an amount by weight of between 7.5 and 450 ppm, with respect to the polyelectrolyte.

9. Absorbent material according to one of the preceding claims, characterised in that the isothiazolin-3-one derivative is combined with a salt of a transition metal.

10. Absorbent material according to claim 9, characterised in that the transition metal is chosen from copper and zinc.

11. Absorbent material according to one of claims 9 or 10, characterised in that the weight ratio of isothiazolin-3-one derivative/transition metal is between 0.012 and 0.018.

12. Use of the absorbent material according to one of the preceding claims, characterised in that it is used as an improved retention additive incorporated in absorbent tampons for body hygiene, in particular those for nappies for babies or for incontinent adults, or for sanitary towels.

13. Process for obtaining an absorbent material according to one of the preceding claims, characterised in that at least one isothiazolin-3-one derivative and the hydrocolloidal polyelectrolyte are first added to a water-alcohol mixture in proportions of between 80/20 and 90/10, and the mixture is then filtered.

14. Process for obtaining an absorbent material according to claim 13, characterised in that a salt of a transition metal is added before the hydrocolloidal polyelectrolyte.